# EUROPEAN PATENT APPLICATION

(11) **EP 2 485 171 A2**
(43) Date of publication of application: **08.08.2012**
(21) Application number: 12153873.0
(22) Date of filing: 03.02.2012
(51) Int. Cl.: G06F 19/00

(54) **Patient helper with egress handle**

(30) Priority: 07.02.2011 US 201161440141 P
(71) Applicant: Hill-Rom Services, Inc., Batesville, IN 47006-9167 (US)
(72) Inventor: Allen, James Maurice, Batesville, IN Indiana 47006 (US)
(74) Representative: Findlay, Alice Rosemary

(57) **Abstract**

A patient support apparatus includes a patient support structure to support a patient, a graphical user interface coupled to the patient support structure, and control circuitry coupled to the graphical user interface. The graphical user interface is operable to display at least one field over which a caregiver's s finger is swiped, without moving any icon, to signal the control circuitry to unlock the graphical user interface for display of a control screen on the graphical user interface for subsequent use by the caregiver in controlling features of the patient support apparatus.

## Description

The present disclosure relates to patient support apparatuses such as hospital beds. More particularly, the present disclosure relates to patient support apparatuses having graphical user interfaces for viewing data and entering commands.

Patient support apparatuses, such as hospital beds, having graphical user interfaces or display screens are known in the art. The graphical user interfaces of hospital beds oftentimes are touch screens that display icons which are used to control functions of the hospital bed or to display information of possible interest to caregivers concerning bed functions and features. See, for example, U.S. Patent Application Publication No. 2008/0235872 Al which is titled "User Interface for Hospital Bed." See also U.S. Patent Application Publication No. 20/8/1172789 A1 which is titled "Patient Support with Improved Control." Inadvertent activation of user inputs of hospital beds is an ongoing concern and ways to mitigate such inadvertent activation is of continuing interest to users of hospital beds and other types of patient support apparatuses.

The present invention comprises one or more of the following features, alone or in any combination:

A patient support apparatus may include a patient support structure to support a patient, a graphical user interface that may be coupled to the patient support structure, and control circuitry that may be coupled to the graphical user interface. The graphical user interface may be operable to display at least one field over which a caregiver's finger may be swiped without moving any icon to signal the control circuitry to unlock the graphical user interface for display of a control screen on the graphical user interface for subsequent use by the caregiver in controlling features of the patient support apparatus.

In some embodiments, the at least one field is oriented horizontally. In other embodiments, the at least one field is oriented vertically. Field orientations other than horizontal and vertical are within the scope of this disclosure. In some embodiments, the at least one field may include a stationary indicia that indicates the direction that the caregiver's finger may be swiped to unlock the graphical user interface. If the field is oriented horizontally, the indicia may include a horizontal arrow and the word "swipe" adjacent the arrow. If the field is oriented vertically, the indicia may include a vertical arrow and the word "swipe" adjacent the arrow. The caregiver's finger may be required to be swiped over substantially an entire length of the fields before the graphical user interface is unlocked.

Alternatively or additionally, at least one graphical indicator may be situated adjacent the field. The at least one graphical indicator may remain stationary and may change in characteristic as the caregiver's finger is swiped over the field. For example, that at least one graphical indicator may include a plurality of graphical indicators that may be spaced apart from each other. The plurality of graphical indicators may comprise radio buttons that may change illumination state as the caregiver's finger is swiped over the field. Thus, the radio buttons may change from a first color to a second color as the caregiver's finger is swiped over the field.

If desired, a room number in which the patient support apparatus is situated may be displayed on the graphical user interface even when the graphical user interface is locked. Alternatively or additionally, a patient identifier of a patient supported on the patient support apparatus may be displayed on the graphical user interface even when the graphical user interface is locked. In some embodiments, after a period of inactivity, the graphical user interface may be operated to display the field in response to the graphical user interface being touched at any location thereon. Thus, the field and graphical indicators, if any, may not be shown on the graphical user interface until the graphical user interface is touched somewhere.

The invention will now be further described by way of example with reference to the accompanying drawings, in which:

Fig. 1 is a perspective view of a hospital bed having a graphical user interface or display screen coupled to a siderail of the hospital bed;

Fig. 2 is a block diagram showing electrical circuitry of the hospital bed in communication with a remote computer;

Fig. 3 is a screen shot of a swipe to unlock screen having a horizontally oriented field over which a caregiver's finger is swiped to unlock other screens that are used to control features of the hospital bed and having a set of graphical indicators above the field to indicate the progress of the caregiver's finger being swiped over the field; and

Fig. 4 is a screen shot of an alternative swipe to unlock screen having a vertically oriented field over which a caregiver's finger is swiped to unlock other screens that are used to control features of the hospital bed and having a set of graphical indicators to the left of the field to indicate the progress of the caregiver's finger being swiped over the field.

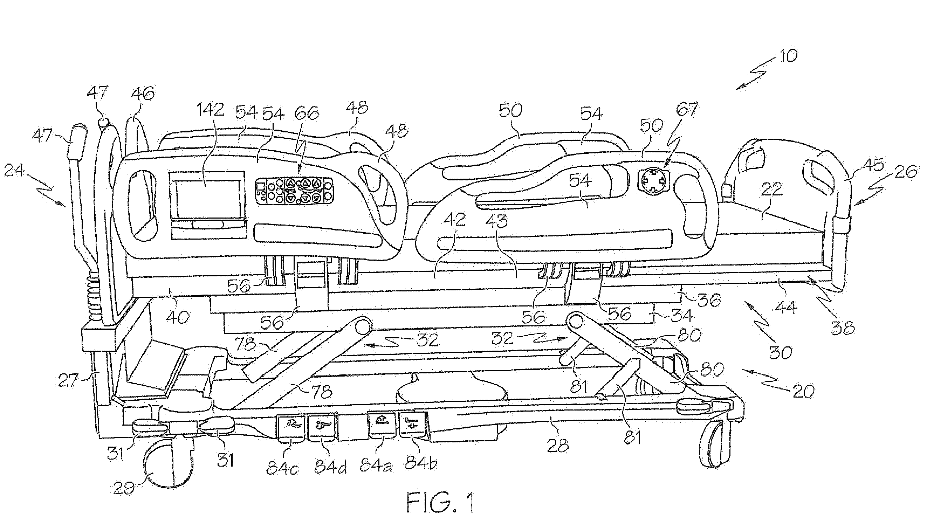

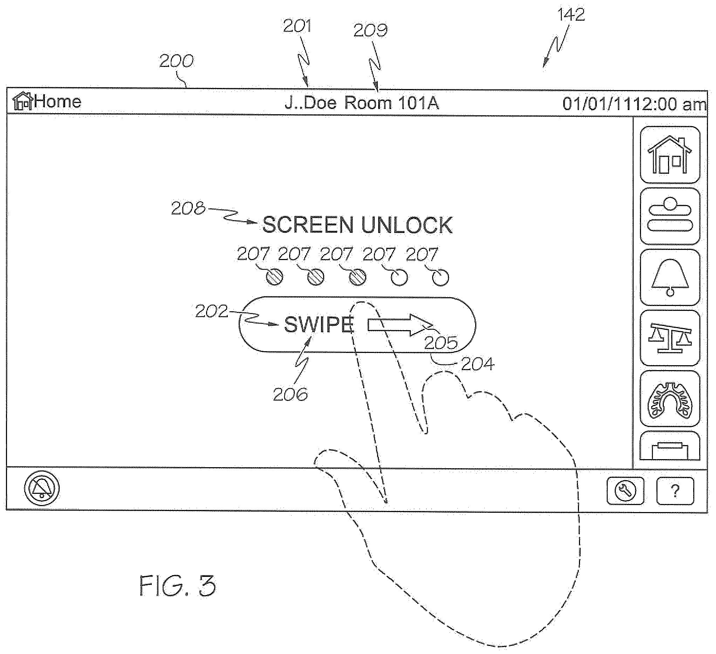

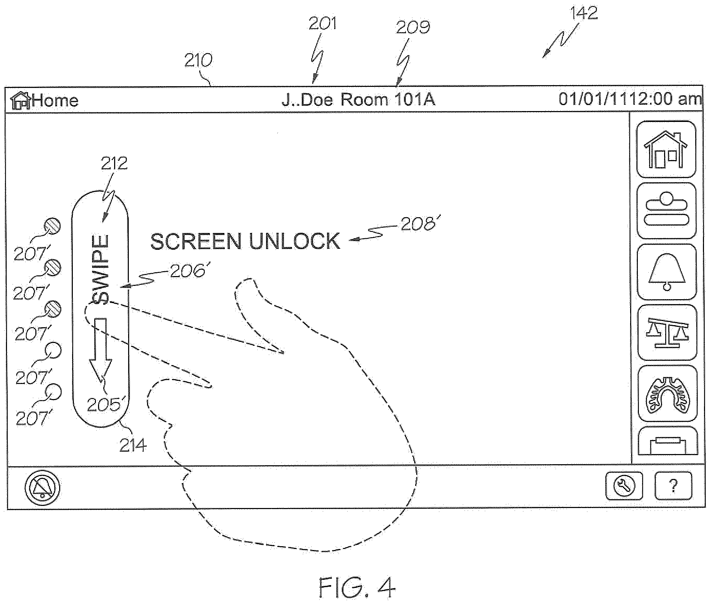

A patient support apparatus, such as illustrative hospital ben 10, includes a patient support structure such as a frame 20 that supports a surface or mattress 22 as shown in Fig. 1. Thus, according to this disclosure a bed frame, a mattress or both are examples of things considered to be within the scope of the term "patient support structure." However, this disclosure is applicable to other types of patient support apparatuses and other patient support structures, including other types of beds, surgical tables, examination tables, stretchers, and the like. As will be described below in connection with Figs. 3 and 4, bed 10 includes a "swipe to unlock" feature that prevents inadvertent or accidental use of a graphical user interface 142 of bed 10. It is contemplated by this disclosure that the "swipe to unlock" feature disclosed herein may be implemented on any type of electronic device having a touch screen input including manufacturing equipment such as machine fools, communication equipment such as hand-held telephone devices, computer equipment such as personal computers and lap tops, electronic reading pads, automotive dashboard controls, and so forth.

Referring again to Fig. 1, frame 20 of bed 10 includes a base 28, an upper frame assembly 30 and a lift system 32 coupling upper frame assembly 30 to base 28. Lift system 32 is operable to raise, lower, and tilt upper frame assembly 30 relative to base 28. Bed 10 has a head end 24 and a foot end 26. Hospital bed 10 further includes a footboard 45 at the foot end 26 and a headboard 46 at the head end 24. Illustrative bed 10 includes a pair of push handles 47 coupled to an upstanding portion 27 of base 28 at the head end 24 of bed 10. Headboard 46 is coupled to upstanding portion 27 of base 28 as well. Footboard 45 is coupled to upper frame assembly 30. Base 28 includes wheels or casters 29 that roll along a floor (not shown) as bed 10 is moved from one location to another. A set of foot pedals 31 are coupled to base 31 and are used to brake and release casters 29.

Illustrative hospital bed 10 has four siderail assemblies coupled to upper frame assembly 30 as shown in Fig. 1. The four siderail assemblies include a pair of head siderail assemblies 48 (sometimes referred to as head rails) and a pair of foot siderail assemblies 50 (sometimes referred to as foot rails). Each of the siderail assemblies 48, 50 is movable between a raised position, as shown in Fig. 1, and a lowered position (not shown). Siderail assemblies 48, 50 are sometimes referred to herein as siderails 48, 50. Each siderail 48, 50 includes a barrier panel 54 and a linkage 56. Each linkage 56 is coupled to the upper frame assembly 30 and is configured to guide the barrier panel 54 during movement of siderails 48, 50 between the respective raised and lowered positions. Barrier panel 54 is maintained by the linkage 56 in a substantially vertical orientation during movement of siderails 48, 50 between the respective raised and lowered positions.

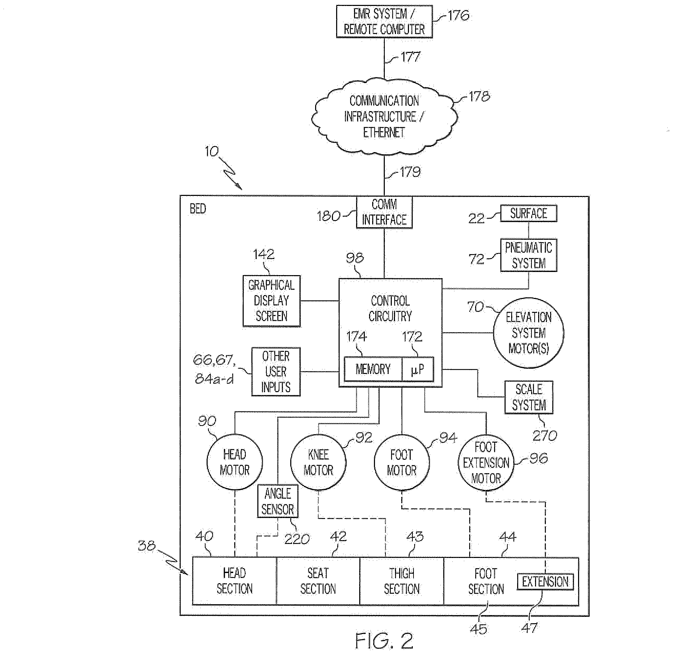

Upper frame assembly 30 includes a lift frame 34, a weigh frame 36 supported with respect to lift frame 34, and a patient support deck 38. Patient support deck 38 is carried by weigh frame 36 and engages a bottom surface of mattress 22. Patient support deck 38 includes a head section 40, a seat section 42, a thigh section 43 and a foot section 44 in the illustrative example as shown in Fig. 1 and as shown diagrammatically in Fig. 2. Sections 40, 43, 44 are each movable relative to weigh frame 36. For example, head section 40 pivotably raises and lowers relative to seat section 42 whereas foot section 44 pivotably raises and lowers relative to thigh section 43. Additionally, thigh section 43 articulates relative to seat section 42. Also, in some embodiments, foot section 44 is extendable and retractable to change the overall length of foot section 44 and therefore, to change the overall length of deck 38. For example, foot section 44 includes a main portion 45 and an extension 47 in some embodiments as shown diagrammatically in Fig. 2.

In the illustrative embodiment, seat section 42 is fixed in position with respect to weigh frame 36 as patient support deck 38 moves between its various patient supporting positions including a horizontal position, shown in Fig. 1, to support the patient in a supine position, for example, and a chair position (not shown) to support the patient in a sitting up position. In other embodiments, seat section 42 also moves relative to weigh frame 36, such as by pivoting and/or translating. Of course, in those embodiments in which seat section 42 translates along upper frame 42, the thigh and foot sections 43, 44 also translate along with seat section 42. As bed 10 moves from the bed position to the chair position, foot section 44 lowers relative to thigh section 43 and shortens in length due to retraction of the extension 47 relative to main portion 45. As bed 10 moves from the chair position to the bed position, foot section 44 raises relative to thigh section 43 and increases in length due to extension of the extension relative to main portion 45. Thus, in the chair position, head section 40 extends upwardly from weigh frame 36 and foot section extends downwardly from thigh section 43.

As shown diagrammatically in Fig. 2, bed 10 includes a head motor or actuator 90 coupled to head section 40, a knee motor or actuator 92 coupled to thigh section 43, a foot motor or actuator 94 coupled to foot section 44, and a foot extension motor or actuator 96 coupled to foot extension 47. Motors 90, 92, 94, 96 may include, for example, an electric motor of a linear actuator. In those embodiments in which seat section 42 translates along upper frame 30 as mentioned above, a seat motor or actuator (not shown) is also provided. Head motor 90 is operable to raise and lower head section 40, knee motor 92 is operable to articulate thigh section 43 relative to seat section 42, foot motor 94 is operable to raise and lower foot section 44 relative to thigh section 43, and foot extension motor 96 is operable to extend and retract extension 47 of foot section 44 relative to main portion 44 of foot section 44.

In some embodiments, bed 10 includes a pneumatic system 72 that controls inflation and deflation of various air bladders or cells (some of which are shown diagrammatically as icons in Figs. 37, 39 and 40) of mattress 22. The pneumatic system 72 is represented in Fig. 2 as a single block but that block 72 is intended to represent one or more air sources (e.g., a fan, a blower, a compressor) and associated valves, manifolds, air passages, air lines or tubes, pressure sensors, and the like, as well as the associated electric circuitry, that are typically included in a pneumatic system for inflating and deflating air bladders of mattresses of hospital beds.

As also shown diagrammatically in Fig. 2, lift system 32 of bed 10 includes one or more elevation system motors or actuators 70, which in some embodiments, comprise linear actuators with electric motors. Thus, actuators 70 are sometimes referred to herein as motors 70. Alternative actuators or motors contemplated by this disclosure include hydraulic cylinders and pneumatic cylinders, for example. The motors 70 of lift system 32 are operable to raise, lower, and tilt upper frame assembly 30 relative to base 28. In the illustrative embodiment, one of motors 70 is coupled to, and acts upon, a set of head end lift arms 78 and another of motors 70 is coupled to, and acts upon, a set of foot end lift arms 80 to accomplish the raising, lowering and tilting functions of upper frame 30 relative to base 28. Guide links 81 are coupled to base 28 and to lift arms 80 in the illustrative example as shown in Fig. 1. Lift system of ben 10 is substantially similar to the lift system of the VERSACARE^{®} bed available from Hill-Rom Company, Inc. Other aspects of bed 10 are also substantially similar to the VERSACARE^{®} bed and are described in more detail in U.S. Patent Nos. 6,658,680; 6,611,979; 6,691,346; 6,957,461; and 7,296,312.

In the illustrative example, bed 10 has four foot pedals 84a, 84b, 84c, 84d coupled to base 28 as shown in Fig. 1. Foot pedal 84a is used to raise upper frame assembly 30 relative to base 28, foot pedal 84b is used to lower frame assembly 30 relative to base 28, foot pedal 84c is used to raise head section 40 relative to frame 36, and foot pedal 84d is used to lower head section 40 relative to frame 36. In other embodiments, foot pedals 84a-d are omitted.

Each siderail 48 includes a first user control panel 66 coupled to the outward side of the associated barrier panel 54 and each siderail 50 includes a second user control panel 67 coupled to the outward side of the associated barrier panel 54. Controls panels 66, 67 include various buttons that are used by a caregiver (not shown) to control associated functions of bed 10. For example, control panel 66 includes buttons that are used to operate head motor 90 to raise and lower the head section 40, buttons that are used to operate knee motor to raise and lower the thigh section, and buttons that are used to operate motors 70 to raise, lower, and tilt upper frame assembly 30 relative to base 28. In the illustrative embodiment, control panel 67 includes buttons that are used to operate motor 94 to raise and lower foot section 44 and buttons that are used to operate motor 96 to extend and retract foot extension 47 relative to main portion 45. In some embodiments, the buttons of control panels 66, 67 comprise membrane switches.

As shown diagrammatically in Fig. 2, bed 10 includes control circuitry 98 that is electrically coupled to motors 90, 92, 94, 96 and to motors 70 of lift system 32. Control circuitry 98 is represented diagrammatically as a shingle block 98 in Fig. 6, but control circuitry 98 in some embodiments comprises various circuit boards, electronics modules, and the like that are electrically and communicatively interconnected. Control circuitry 98 includes one or more microprocessors 172 or microcontrollers that execute software to perform the various control functions and algorithms described herein. Thus, circuitry 98 also includes memory 174 for storing software, variables, calculated values, and the like as is well known in the art.

As also shown diagrammatically in Fig. 2, a user inputs block represents the various user inputs such as buttons of control panels 66, 67 and pedals 84a-d, for example, that are used by the caregiver or patient to communicate input signals to control circuitry 98 of bed 10 to command the operation of the various motors 70, 90, 92, 94, 96 of bed 10, as well as commanding the operation of other functions of bed 10. Bed 10 includes at least one graphical user input or display screen 142 coupled to a respective siderail 48 as shown in Fig. 1. Display screen 142 is coupled to control circuitry 98 as shown diagrammatically in Fig. 2. In some embodiments, two graphical user interfaces 142 are provided and are coupled to respective siderails 48. Alternatively or additionally, one or more graphical user interfaces are coupled to siderails 50 and/or to one or both of the headboard 46 and footboard 45. Thus, it is contemplated by this disclosure that a graphical user interface 142 may be coupled to any of barriers 45, 46, 48, 50 of bed 10. Alternatively or additionally, graphical user interface 142 is provided on a hand-held device such as a pod or pendant that communicates via a wired or wireless connection with control circuitry 98.

Control circuitry 98 receives user input commands from graphical display screen 142 when display screen 142 is activated. The user input commands control various functions of bed 10 such as controlling the pneumatic system 72 and therefore, the surface functions of surface 22. In some embodiments, the input commands entered on user interface 142 also control the functions of one or more of motors 70, 90, 92, 94, 96 but this need not be the case. In some embodiments, input commands entered on the user interface 142 also control functions of a scale system 270, which is discussed in more detail below.

Various examples of the various alternative or additional functions of bed 10 that are controlled by display screen 142 in various embodiments can be found in U.S. Patent Application Publication Nos. 2008/0235872 A1 and 2008/0172789 A1 and in U.S. Application No. 13/249,336, filed September 30, 2011, and titled "Hospital Bed with Graphical User Interface Having Advanced Functionality," each of which is hereby incorporated by reference herein. According to this disclosure, control circuitry 98 is configured to deactivate display screen 142 if screen 142 has not been used to control a function of bed 10 within a threshold amount of time, such as 30 seconds to 5 minutes, for example. Once display screen 142 has been deactivated, inadvertent or accidental activation of display screen 142, as well as inadvertent or accidental activation of functions of bed 10 via use of display screen 142, is prevented because a user is required to perform a certain touch and swipe sequence on display screen 142 to re-activate it as is discussed below in connection with Figs. 3 and 4.

In some embodiments, control circuitry 98 of bed 10 communicates with a remote computer device 176 via communication infrastructure 178 such as an Ethernet of a healthcare facility in which bed 10 is located and via communications links 177, 179 as shown diagrammatically in Fig. 2. Computer device 176 is sometimes simply referred to as a "computer" herein. Remote computer 176 may be part of an electronic medical records (EMR) system, for example. However, it is within the scope of this disclosure for circuitry 98 of bed 10 to communicate with other computers such as those included as part of a nurse call system, a physician ordering system, an admission/discharge/transfer (ADT) system, or some other system used in a healthcare facility in other embodiments. Ethernet 178 in Fig. 2 is illustrated diagrammatically and is intended to represent all of the hardware and software that comprises a network of a healthcare facility.

In the illustrative embodiment, bed 10 has a communication interface or port 180 which provides bidirectional communication via link 179 with infrastructure 178 which, in turn, communicates bidirectionally with computer 176 via link 177. Link 179 is a wired communication link in some embodiments and is a wireless communications link in other embodiments. Thus, communications link 179, in some embodiments, comprises a cable connects bed 10 to a wall mounted jack that is included as part of a bed interface unit (BIU) or a network interface unit (NIU) of the type shown and described in U.S. Patent Noes. 7,538,659 and 7,319,386 and in U.S. Patent Application Publication Nos. 2009/0217080 A1, 2009/0212925 A1 and 2009/0212926 A1, each of which are hereby expressly incorporated by reference herein. In other embodiments, communications link 179 comprises wireless signals sent between bed 10 and a wireless interface unit of the type shown and described in U.S. Patent Application Publication No. 2007/0210917 A1. Communications link 177 comprises one or more wired links and/or wireless links as well, according to this disclosure.

According to one embodiment, a swipe to unlock screen 200 is displayed on graphical user interface 142 sand includes a field or bar 204 that extends horizontally. Field 204 includes a stationary indicia or graphic 202. In the illustrative example, indicia 202 includes an arrow icon 205 and the text "swipe" 206 adjacent icon 205. Thus, indicia 202 indicates the action that a caregiver needs to perform to unlock or re-activate graphical user interface 142. Field 204 extends a substantial amount across a horizontal dimension of the graphical user interface 142, such as extending at least a fourth or a third of the way across the horizontal dimension of screen 142.

A set of graphical indicators 207 are situated above and adjacent to field 204 and the text "screen unlock" 207 appears above and adjacent to the set of graphical indicators 207. To re-activate graphical user interface 142 for use in commanding bed functions, a caregiver touches field 204 near the left hand side of field 204 and swipes his or her finger to the right in the illustrative example of Fig. 3. Alternatively, icon 202 indicates that a person should swipe from right to left. However, the illustrative example is more ergonomic for right handed persons. In the illustrative example of Fig. 3, a caregiver's right hand is shown in phantom and has moved approximately half way over or along field 204.

As the caregiver's finger is swiped over field 204, an illumination state of each of graphical indicators 207 is changed to indicate the progress of the caregiver's finger along the field. For example, the illumination state may change by changing color or by appearing empty and then full. After successfully swiping over or along bar 204 from the left hand side to the right hand side of bar 204, display screen 142 is unlocked or re-activated and a home screen or main screen appears on display screen 142.

In one embodiment, prior to a caregiver swiping across field 204 indicators 207 are filled in with the same background color as the overall color of screen 200 (e.g., black or dark blue) and as the caregiver's finger passes beneath each of the indicators 207, the indicator color is changed to another color (e.g., green or red). In the illustrative example, the caregiver's finger has passed beneath three of indicators 207 and has two more to go. It will be appreciated therefore, that in order to unlock graphical user interface 142 to command bed operations, the caregiver is required to swipe his or her finger over substantially an entire length of field 204.

In some embodiments, bar 204, indicia 202, indicators 207, and text 208 do not appear on the graphical user interface 142 until interface 142 is first touched somewhere thereon. Alternatively or additionally, a button somewhere else, such as on a siderail 48, 50 of bed 10 is touched to cause bar 204, indicia 202, indicators 207, and text 208 to appear on display screen 142. Furthermore, after a period of inactivity, bar 204, indicia 202, indicators 207, and text 208 will disappear from display screen 142 in some embodiments. In the illustrative example, a patient identifier 201 and a room identifier 209 are shown at the top of screen 200. The patient identifier 201 indicates the patient that is assigned to bed 10 and therefore, will change as different patient's are assigned. The room identifier 209 indicates the room in which bed 10 is located. In some embodiments, identifiers 201 and 209 continue to appear on screen 200 even when bar 204, indicia 202, indicators 207, and text 208 do not appear and the graphical user interface 142 is locked from use.

According to another embodiment, a swipe to unlock screen 210 includes a vertically oriented field 214 and a stationary indicia 212 an icon or graphic 212 situated in a top region of a bar 214 that extends vertically as shown in Fig. 4. Field 214 includes a stationary indicia or graphic 212. In the illustrative example, indicia 212 includes an arrow icon 205' and the text "swipe" 206 adjacent icon 205'. Thus, indicia 212 indicates the action that a caregiver needs to perform to unlock or re-activate graphical user interface 142. Field 214 extends a substantial amount along the vertical dimension of graphical user interface 142 such as extending more than half way down the vertical dimension of the graphical user interface 142.

To re-activate interface 142 for use in commanding bed function, a caregiver touches field 214 near the top and swipes his or her finger downwardly substantially all the way down to a bottom of field 214. The text "screen unlock" 208' appears adjacent to and to the right of bar 214 on screen 210 and a set of graphical indicators 207' appear adjacent to and to the left of bar 214 in the Fig. 4 example. The illumination state of indicators 207' change as the caregiver's finger passes by them while the caregiver is swiping downwardly. The operation and features of indicators 207' are substantially the same as indicators 207 described above and therefore, do not need to be repeated in connection with Fig. 4. Also, identifiers 201 and 209 also appear on screen 210 in the same manner as described above with regard to screen 200. Furthermore, after a period of inactivity bar 214, indicia 212, indicators 207' and text 208' disappear from the graphical user interface 142 in some embodiments. Touching the display screen 142 or another button somewhere else return them to the display screen 142.

Data regarding the status and use of screens 200, 210 is transmitted from bed 10 to remote computer 176 in some embodiments. For example, computers 176 may store information about the date and time at which the display screen 142 becomes deactivated and the date and time at which a user successfully swipes over field 204 or field 214 to re-activate display screen 142. Information regarding unsuccessful attempts to unlock or re-activate display screen 142 for use may also be transmitted from bed 10 to the remote computer 176 in some embodiments. Identifiers 201 and 209 are transmitted to bed from computer 176 for display in some embodiments.

Another type of bed having a display screen, similar to illustrative display screen 142, with associated control circuitry programmed with a "swide to unlock" feature is shown and described in U.S. Application Noes. 12/891,909, filled September 28, 2010 and titled "Hospital Bed with Chair Loelcouf'anct 12/957,491, filed December 1,2010 and titled "Removable Integrated Board and Partial Foot Section." This alternative bed is in development currently under the project name "Series 8."

The "swipe to unlock" feature disclosed here is unlike the "slide to unlock" feature disclosed in U.S. Pat. Nows. 8,046,721 and 7,657,849. Those patents disclose an unlock image or icon that is moved along a path. Such a moving unlock image or icon is absent from the disclosed "swipe to unlock" embodiments disclosed herein, such as the example of Figs. 3 and 4. That is, no image or icon in fields 204, 214 moves as the caregiver's finger swipes over or along those fields 204, 214. The graphical indicators 207, 207' don't move either, rather, they just change illumination state.

In alternative embodiments, in lieu of horizontal field 204 or vertical field 214, an inclined or angled field is provided on graphical user interface 142. In the Fig. 3 and 4 examples discussed above, fields 204, 214 each define a generally straight or linear path over which a caregiver is to swipe his or her finger. In other embodiments, a non-linear field is provided. For example, fields that define a Z-shaped path, a W-shaped path, an L-shaped path, a C-shaped, U-shaped path, an O-shaped (or circular) path, and so on are contemplated by this disclosure. These paths may be at different orientations than the above-listed latters ordinarily appear (e.g., inverted or rotated L, mirror image C, upside down U, just to name a few). In the case of an O-shaped or circular path, a set starting point from which a caregiver is to swipe and a set direction (e.g., clockwise or counterclockwise) to swipe may be indicated. In such an embodiment, a set ending point is provided adjacent the starting paint. In other embodiments of an O-shaped path, the caregiver has the option of starting anywhere along the path and/or rotating in any direction around the path, as long as a substantially full swipe (e.g., substantially 360 degrees around a circle) is made. In each of the non-linear path alternatives, it is contemplated that indicators similar to indicators 207, 207' are provided along the path to provide visual feedback to the caregiver as to his or her progress of swiping along the path.

Although certain illustrative embodiments have been described in detail above, many embodiments, variations and modifications are possible.

## Claims

1. A patient support apparatus comprising a patient support structure to support a patient,
a graphical user interface coupled to the patient support structure, and control circuitry coupled to the graphical user interface, the graphical user interface being operable to display at least one field over which a caregiver's finger is swiped, without moving any icon, to signal the control circuitry to unlock the graphical user interface for display of a control screen on the graphical user interface for subsequent use by the caregiver in controlling features of the patient support apparatus.

2. The patient support apparatus of claim 1, wherein the at least one field is oriented horizontally.

3. The patient support apparatus of claim 2, wherein the at least one field includes a stationary indicia that indicates the direction that the caregiver's finger should be swiped to unlock the graphical user interface.

4. The patient support apparatus of claim 3, wherein the indicia includes a horizontal amow and word "swipe" adjacent the arrow.

5. The patient support apparatus of claim, 1, wherein the at least one field is oriented vertically.

6. The patient support apparatus of claim 5, wherein the at least one field includes a stationary indicia that indicates the direction that the caregiver's finger should be swiped to unlock the graphical user interface.

7. The patient support apparatus of claim 6, wherein the indicia includes a vertical arrow and word "swipe" adjacent the arrow.

8. The patient support apparatus of any preceding claim, further comprising at least one graphical indicator situated adjacent the field, the at least one graphical indicator remaining stationary and changing in characteristic as the caregiver's finger is swiped over the field.

9. The patient support apparatus of claim 8, wherein the at least one graphical indicator includes a plurality of graphical indicators that are spaced apart from each other.

10. The patient support apparatus of claim 9, wherein the plurality of graphical indicators comprise radio buttons that change illumination state as the caregiver's finger is swiped over the field,

11. The patient support apparatus of claim 9, wherein the plurality of graphical indicators comprise radio buttons that change from a first color to a second color as the caregiver's finger is swiped over the field.

12. The patient support apparatus of any preceding claim, wherein a room number in which the patient support apparatus is situated is displayed on the graphical user interface even when the graphical user interface is locked.

13. The patient support apparatus of any preceding claim, wherein a patient identifier of a patient supported on the patient support apparatus is displayed on the graphical user interface even when the graphical user interface is locked.

14. The patient support apparatus of any preceding claim, wherein after a period of inactivity, the graphical user interface is operated to display the field in response to the graphical user interface being touched at any location thereon.

15. The patient support apparatus of any preceding claim, wherein the caregiver finger is required to be swiped over substantially an entire length of the field before the graphical user interface is unlocked.
